# DEMANDE DE BREVET EUROPEEN

(11) **EP 1 537 853 A1**
(43) Date de publication de la demande: **08.06.2005**
(21) Numéro de dépôt: 04292744.2
(22) Date de dépôt: 19.11.2004
(51) Int. Cl.: A61K 7/13

(54) **Utilisation pour la coloration des fibres kératiniques d'une composition comprenant un composé comportant un cycle lactone et/ou le colorant correspondant à ce composé dont le cycle lactone est ouvert**

(30) Priorité: 21.11.2003 FR 0350884
(71) Demandeur: L'OREAL, 75008 Paris (FR)
(72) Inventeur: Plos, Grégory Gluck Heim, 158-0097 Tokyo (JP); Gawtrey, Jonathan, 92100 Boulogne (FR); Kravtchenko, Sylvain, 92600 Asnieres (FR)
(74) Mandataire: Fevrier, Murielle

(57) **Abrégé**

La présente invention a pour objet l'utilisation pour la coloration des fibres kératiniques, et en particulier des fibres kératiniques humaines telles que les cheveux, d'une composition comprenant au moins un composé comportant un cycle lactone et / ou le colorant correspondant à ce composé dont le cycle lactone est ouvert.

La présente invention permet en particulier d'obtenir une coloration des fibres kératiniques avec des reflets intenses et tenaces pouvant être modifiée, effacée et reformée plusieurs fois sans perte de couleur.

## Description

La présente invention a pour objet l'utilisation pour la coloration des fibres kératiniques, et en particulier des fibres kératiniques humaines telles que les cheveux, d'une composition comprenant au moins un composé comportant un cycle lactone et 1 ou le colorant correspondant à ce composé dont le cycle lactone est ouvert.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques, et en particulier les fibres kératiniques humaines telles que les cheveux, avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, azoïque cationique, xanthénique, acridinique azinique, triarylméthane, benzénique nitré ou des colorants naturels.

L'utilisation des colorants directs est très répandue car ils présentent certains avantages par rapport aux précurseurs de colorants d'oxydation et, notamment, une diminution des risques potentiels d'allergie, l'absence de sensibilisation du cheveu due au processus oxydatif et des temps de pose moins longs.

Les colorations obtenues sont cependant temporaires ou semi-permanentes, car la nature des interactions qui lient les colorants directs à la fibre kératinique, et leur désorption de la surface et / ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

L'utilisateur peut choisir le mode de coloration lui permettant d'obtenir des nuances adaptées à ses besoins en terme de reflets et de ténacité. Cependant, pour effacer les couleurs ainsi obtenues, il doit utiliser des compositions de décoloration comprenant un ou plusieurs agents oxydants ou des réducteurs de type réductone, thiol ou sulfite. Parmi les agents oxydants classiquement utilisés, on peut citer le peroxyde d'hydrogène ou des composés susceptibles de produire du peroxyde d'hydrogène par hydrolyse, comme par exemple le peroxyde d'urée ou les persels tels que les perborates, les persulfates, les percarbonates. L'utilisation de ces agents oxydants présente l'inconvénient d'entraîner une dégradation non négligeable des fibres kératiniques et d'altérer leurs propriétés cosmétiques. Les cheveux ont tendance à devenir rèches, plus difficilement démêlables et plus fragiles. Les réducteurs de type réductone, thiol ou sulfite présente quant à eux l'inconvénient de ne pas convenir à tous les types de colorants. Si ils permettent d'effacer efficacement et sans endommager la fibre kératinique les colorants azoïques, ils sont inopérants avec les colorants dérivés des anthraquinones.

Le but de la présente invention est de fournir de nouvelles compositions pour la coloration des fibres kératiniques qui ne présentent pas les inconvénients de celles de l'art antérieur. En particulier, le but de la présente invention est de fournir de nouvelles compositions pour la coloration des fibres kératiniques permettant d'obtenir rapidement des colorations avec des reflets intenses et tenaces qui peuvent s'effacer grâce à un agent extérieur qui n'altère pas les fibres kératiniques comme un agent de pH ou la chaleur par exemple.

Ce but est atteint avec la présente invention qui a pour objet l'utilisation pour la coloration des fibres kératiniques, et en particulier des fibres kératiniques humaines telles que les cheveux, d'une composition comprenant, dans un milieu approprié pour la teinture, au moins un composé choisi parmi les composés comportant un cycle lactone de formule (I); les colorants correspondant aux composés de formule (I) dont le cycle lactone est ouvert et leurs sels d'addition : 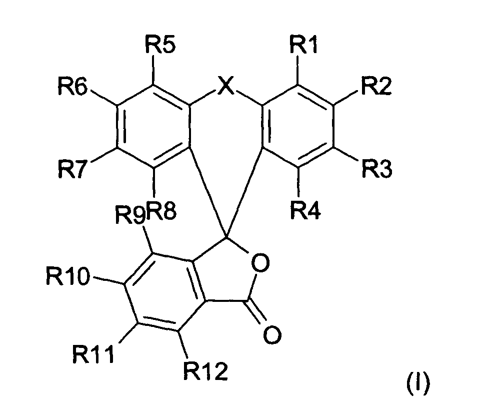 dans laquelle :
- R₁, R₃, R₄, R₅, R₆, R₇, R₈, R₉, R₁₀, R₁₁ et R₁₂ représentent, indépendamment l'un de l'autre :
   - un atome d'hydrogène ;
   - un radical halogéno ;
   - un radical hydroxyle ;
   - un radical nitro ;
   - un radical amino ;
   - un radical carboxy ;
   - un radical aminocarbonyle ;
   - un radical cyano ;
   - un radical issu d'une chaîne hydrocarbonée comportant de 1 à 100 atomes de carbone, de préférence 1 à 50, linéaire ou ramifiée, acyclique ou mono ou polycyclique, condensée ou non condensée, saturée ou insaturée, aromatique ou non aromatique, pouvant être interrompue par un ou plusieurs hétéroatomes choisis parmi les atomes d'azote, d'oxygène et de soufre ou par un ou plusieurs groupements carbonyle, pouvant être terminée par un groupement hydrogénocarbonyle ou par un groupement contenant un ou plusieurs hétéroatomes choisis parmi les atomes d'azote, d'oxygène et de soufre et pouvant être substituée par un ou plusieurs groupements choisis parmi les radicaux hydroxyle, halogéno, carboxy, carboxyalkyle en C₁-C₉, cyano, amino ;
   les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₆-C₁₂ ; un radical aryle en C₆-C₁₈ ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ;
- R₂ représente :
   - un atome d'hydrogène ;
   - un radical halogéno ;
   - un radical nitro ;
   - un radical amino ;
   - un radical carboxy ;
   - un radical aminocarbonyle ;
   - un radical cyano ;
   - un radical issu d'une chaîne hydrocarbonée comportant de 1 à 100 atomes de carbone, de préférence 1 à 50, linéaire ou ramifiée, acyclique ou mono ou polycyclique, condensée ou non condensée, saturée ou insaturée, aromatique ou non aromatique, pouvant être interrompue par un ou plusieurs hétéroatomes choisis parmi les atomes d'azote, d'oxygène et de soufre ou par un ou plusieurs groupements carbonyle, pouvant être terminée par un groupement hydrogénocarbonyle ou par un groupement contenant un ou plusieurs hétéroatomes choisis parmi les atomes d'azote, d'oxygène et de soufre et pouvant être substituée par un ou plusieurs groupements choisis parmi les radicaux hydroxyle, halogéno, carboxy, carboxyalkyle en C₁-C₉, cyano, amino ;
   les radicaux amino étant non substitués ou substitués par un ou deux radicaux, identiques ou différents, choisis parmi un radical alkyle en C₁-C₉ substitué ; un radical hydroxyalkyle en C₁-C₉ ; un radical alcényle en C₂-C₉ ; un radical cycloalkyle en C₆-C₁₂ ; un radical aryle en C₆-C₁₈ non substitué ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ;
- deux des radicaux R₁, R₂, R₃, R₄, R₅, R₆, R₇, R₈, R₉, R₁₀, R₁₁ et R₁₂ portés par deux atomes de carbone adjacents pouvant former ensemble un groupement mono ou polycarbocyclique aromatique, condensé ou non condensé, comprenant de 5 à 20 chaînons, un ou plusieurs atomes de carbone pouvant être remplacés par un atome d'oxygène, d'azote, de soufre ou de phosphore, non substitué ou substitué par un ou plusieurs atomes d'halogène, un ou plusieurs radicaux hydroxyle, amino, carboxy, aryle en C₆-C₁₈, cyano ;
   les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₆-C₁₂ ; un radical aryle en C₆-C₁₈ ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ;
- X représente un atome de carbone, d'azote, de soufre, d'oxygène, de phosphore.

La présente invention a également pour objet un procédé de traitement des fibres kératiniques, et en particulier des fibres kératiniques humaines telles que les cheveux, mettant en oeuvre la composition conforme à l'invention.

La présente invention permet en particulier d'obtenir rapidement une coloration des fibres kératiniques avec des reflets intenses et tenaces pouvant être effacée puis reformée tout aussi rapidement. Le procédé d'effaçage et de reformation de la couleur peut être reproduit au moins une fois sans perte substantielle de couleur à l'aide d'un agent de pH ou en agissant sur la température.

Les composés de formule (I) comportent un cycle lactone pouvant subir une ouverture en formant un groupement acide en présence de protons. Ces composés sont incolores ou faiblement colorés et les composés correspondant à l'ouverture du cycle lactone sont des espèces colorées et colorantes. En milieu aqueux, il s'établit un équilibre entre les espèces colorées et les espèces non colorées qui dépend du pH et de la température.

Lorsque la composition comprend plusieurs composés choisis parmi les composés de formule (I), les colorants correspondant aux composés de formule (I) dont le cycle lactone est ouvert et leurs sels d'addition, la coloration des fibres kératiniques peut aussi être modifiée à l'aide d'un agent de pH ou en agissant sur la température.

Dans le cadre de la présente invention, on entend par radical alkyle (alk) un radical linéaire ou ramifié, par exemple un radical méthyle, éthyle, n-propyle, iso-propyle, n-butyle ou ter-butyle. Sauf indication contraire, ce radical peut être substitué ou non substitué. Un radical alcoxy est un radical alk-O-, un radical alkylthio est un radical alk-S-, un radical mono ou dialkylamino est un radical -N(alk)ₙ avec n = 1 ou 2, un radical alkylcarbonyle est un radical alk-CO-, un radical alcoxycarbonyle est un adical alk-O-CO-, un radical alkylcarbonylalkyle est un radical alk-CO-alk-, un radical alkylcarbonylamino est un radical alk-CO-NH-, dans chacune de ces définitions le radical alkyle étant tel que défini précédemment.

On entend par radical alcényle, un alkyle de 2 à 10 atomes de carbone et comprenant une ou plusieurs doubles et / ou triples liaisons, conjuguées ou non, substitué ou non substitué.

On entend par radical cycloalkyle un radical alkyle dans lequel les atomes de carbone forment un cycle, par exemple un radical cyclohexyle. Ce radical peut être substitué ou non substitué. Un radical mono ou dicycloalkylamino est un radical amino substitué par un ou deux radicaux cycloalkyle.

On entend par radical aryle (ar) un radical carboné dérivé des composés benzéniques condensés ou non condensés, par exemple phényle, anthracényle ou naphtyle. Sauf indication contraire, ce radical peut être substitué ou non substitué. Un radical mono ou diarylamino est un radical amino substitué par un ou deux radicaux aryle. Un radical mono ou di(arylalkyl)amino est un radical amino substitué par un ou deux radicaux arylalkyle. Un radical arylalkyle est un radical alkyle substitué par un radical aryle. Un radical arylalcoxy est un radical alcoxy substitué par un radical aryle. Un radical arylcarbonyle est un radical ar-CO-, avec ar étant tel que défini précédemment.

On entend par « chaîne hydrocarbonée pouvant être interrompue par un ou plusieurs hétéroatomes » une chaîne hydrocarbonée comportant un ou plusieurs hétéroatomes entre deux atomes de carbone, par exemple un radical alcoxyalkyle.

On entend par « chaîne hydrocarbonée pouvant être terminée par un groupement hydrogénocarbonyle ou par un groupement contenant un ou plusieurs hétéroatomes » une chaîne hydrocarbonée dont l'atome de carbone le plus éloigné de l'atome de carbone relié au noyau aromatique sur lequel est fixée la chaîne porte un groupement hydrogénocarbonyle ou un groupement contenant un ou plusieurs hétéroatomes, par exemple un radical hydrogénocarbonylalkyle.

Un radical halogéno désigne un atome d'halogène choisi parmi le chlore, le brome, l'iode et le fluor.

Dans le cadre de la présente invention, le terme "condensé" signifie au moins deux cycles accolés présentant au moins deux atomes communs.

Un groupement mono ou polyhétérocyclique, condensé ou non condensé, aromatique ou non aromatique, comprenant de 5 à 50 chaînons, peut être par exemple un cycle thiophène, benzofurane, benzothiophène, indole, bispyridine, benzopyrane, quinoline, pyrazole, pyridine, pyrrole, furane, imidazole, benzimidazole.

Un groupement mono ou polycarbocyclique aromatique, condensé ou non condensé, comprenant de 5 à 50 chaînons, un ou plusieurs atomes de carbone pouvant être remplacés par un atome d'oxygène, d'azote, de soufre ou de phosphore peut être par exemple un cycle benzène, naphtalène, anthracène, pyridine, quinoline, thiofène, pyrimidine.

Un radical imino est un radical HN=C.

Un radical arylimino peut être par exemple un radical phénylimino.

Un radical alcoxycarbonylalkylamino est un radical amino substitué par un radical alcoxycarbonylalkyle.

Un radical α-naphtylalkylamino est un radical amino substitué par un radical α-naphtylalkyle, qui est un radical alkyle substitué par un radical α-naphtyle.

Selon un mode de réalisation particulier de l'invention, le composé de formule (I) est tel que :
• R₁, R₃, R₄, R₅, R₆, R₇, R₈, R₉, R₁₀, R₁₁ et R₁₂ représentent, indépendamment l'un de l'autre :
   - un atome d'hydrogène ;
   - un radical halogéno ;
   - un radical hydroxyle ;
   - un radical alkyle en C₁-C₉ ;
   - un radical cycloalkyle en C₆-C₁₂ ;
   - un radical aryle en C₆-C₁₈ ;
   - un radical alcoxy en C₁-C₉ ;
   - un radical alkylthio en C₁-C₉ ;
   - un radical amino ;
   - un radical mono ou dialkyl(C₁-C₉)amino ;
   - un radical mono ou dicycloalkyl(C₆-C₁₂)amino ;
   - un radical mono ou diaryl(C₆-C₁₈)amino ;
   - un radical mono ou di(aryl(C₆-C₁₈)alkyl(C₁-C₉))amino ;
   - un radical N,N-(cycloalkyl(C₆-C₁₂))(alkyl(C₁-C₉))amino ;
   - un radical N,N-(aryl(C₆-C₁₈))(alkyl(C₁-C₉))amino ;
   - un radical N,N-(aryl(C₆-C₁₈)carbonyl)(alkyl(C₁-C₉))amino ;
   - un radical N,N-(alkyl(C₁-C₉)carbonyl)(aryl(C₆-C₁₈))amino ;
   - un radical alkyl(C₁-C₆)carbonylalkyl(C₁-C₆) ;
   - un radical alkyle en C₁-C₆ substitué par un groupement mono ou polyhétérocyclique, condensé ou non condensé, aromatique ou non aromatique, comprenant de 5 à 50 chaînons ;
   - un groupement mono ou polyhétérocyclique, condensé ou non condensé, aromatique ou non aromatique, comprenant de 5 à 50 chaînons ;
      les groupements hétérocycliques pouvant être substitués par un ou plusieurs radicaux hydroxyle, halogéno, amino, alcoxy en C₁-C₉ ;
   - un radical aryl)(C₆-C₁₈)alcoxy(C₁-C₉) ;
   - un radical (alkyl(C₁-C₉)carbonyl)amino ;
   - un radical aminoaryl(C₆-C₁₈) amido ;
   - un radical aminoalcényle en C₂-C₉ ;
   - un radical aryl(C₆-C₁₈)imino ;
   - un radical aryl(C₆-C₁₈)alcényl(C₂-C₉)imino ;
   - un radical alcényl(C₂-C₉)imino ;
   - un radical hydrogénocarbonylalcoxy(C₁-C₉) ;
   les groupements alkyle ou alcényle étant non substitués ou substitués par un ou plusieurs groupements hydroxyle, halogéno, amino, cyano, thio, alcoxy en C₁-C₉, styryle, tétrahydrofuryle, carboxy ou alcoxycarbonyle en C₁-C₉ et les groupements cycloalkyle ou aryle étant non substitués ou substitués par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle ;
• R₂ représente :
   - un atome d'hydrogène ;
   - un radical halogéno ;
   - un radical alkyle en C₁-C₉ ;
   - un radical cycloalkyle en C₆-C₁₂ ;
   - un radical aryle en C₆-C₁₈ ;
   - un radical alcoxy en C₁-C₉ ;
   - un radical alkylthio en C₁-C₉ ;
   - un radical amino ;
   - un radical mono ou dicycloalkyl(C₆-C₁₂)amino ;
   - un radical mono ou di(aryl(C₆-C₁₈)alkyl(C₁-C₉))amino ;
   - un radical alkyl(C₁-C₆)carbonylalkyl(C₁-C₆) ;
   - un radical alkyle en C₁-C₆ substitué par un groupement mono ou polyhétérocyclique, condensé ou non condensé, aromatique ou non aromatique, comprenant de 5 à 50 chaînons ;
   - un groupement mono ou polyhétérocyclique, condensé ou non condensé, aromatique ou non aromatique, comprenant de 5 à 50 chaînons ;
      les groupements hétérocycliques pouvant être substitués par un ou plusieurs radicaux hydroxyle, halogéno, amino, alcoxy en C₁-C₉ ;
   - un radical aryl(C₆-C₁₈)alcoxy(C₁-C₉) ;
   - un radical (alkyl(C₁-C₉)carbonyl)amino ;
   - un radical aminoaryl(C₆-C₁₈) amido ;
   - un radical aminoalcényle en C₂-C₉ ;
   - un radical ary)(C₆-C₁₈)imino ;
   - un radical aryl(C₆-C₁₈)alcényl(C₂-C₉)imino ;
   - un radical alcényl(C₂-C₉)imino ;
   - un radical hydrogénocarbonylalcoxy(C₁-C₉) ;
      les groupements alkyle ou alcényle étant non substitués ou substitués par un ou plusieurs groupements hydroxyle, halogéno, amino, cyano, thio, alcoxy en C₁-C₉, styryle, tétrahydrofuryle, carboxy ou alcoxycarbonyle en C₁-C₉ et les groupements cycloalkyle ou aryle étant non substitués ou substitués par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle
   - un radical N,N-(aryl(C₆-C₁₈)carbonyl)carbonyl(C₁-C₉))amino ;
      le groupement alkyle étant substitué par un ou plusieurs groupements hydroxyle, halogéno, amino, cyano, thio, alcoxy en C₁-C₉, styryle, tétrahydrofuryle, carboxy ou alcoxycarbonyle en C₁-C₉ et le groupement aryle étant non substitué ou substitué par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle ;
   - un radical N,N-(aryl(C₆-C₁₈))(alkyl(C₁-C₉))amino ;
   - un radical mono ou dialkyl(C₁-C₉)amino ;
   - un radical mono ou diaryl(C₆-C₁₈)amino ;
   - un radical N,N-(cycloalkyl(C₆-C₁₂))(alkyl(C₆-C₉))amino ;
      les groupements alkyle étant substitués par un ou plusieurs groupements hydroxyle, halogéno, amino, cyano, thio, alcoxy en C₁-C₉, styryle, tétrahydrofuryle, carboxy ou alcoxycarbonyle en C₁-C₉; le groupement cycloalkyle étant non substitué ou substitué par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle ; les groupements aryle étant non substitués ;
   - un radical N,N-(a)ky)(C₁-C₉)carbonyl)(aryl(C₆-C₁₈))amino ;
      le groupement alkyle étant non substitué ou substitué par un ou plusieurs groupements hydroxyle, halogéno, amino, cyano, thio, alcoxy en C₁-C₉, styryle, tétrahydrofuryle, carboxy ou alcoxycarbonyle en C₁-C₉ et le groupement aryle étant non substitué ;
• deux des radicaux R₁, R₂, R₃, R₄, R₅, R₆, R₇, R₈, R₉, R₁₀, R₁₁ et R₁₂ portés par deux atomes de carbone adjacents pouvant former ensemble un groupement mono ou polycarbocyclique aromatique, condensé ou non condensé, comprenant de 5 à 20 chaînons, un ou plusieurs atomes de carbone pouvant être remplacés par un atome d'oxygène, d'azote, de soufre ou de phosphore, non substitué ou substitué par un ou plusieurs atomes d'halogène, un ou plusieurs radicaux hydroxyle, amino, mono ou dialkyl(C₁-C₄)amino, carboxy, alcoxy(C₁-C₉)carbonylalkyl(C₁-C₉)amino, α-naphtylalkylamino ;
• X représente un atome de carbone, d'azote, de soufre, d'oxygène, de phosphore.

Selon un mode de réalisation préféré de l'invention, le composé de formule (I) est tel que :
- R₁, R₃, R₄, R₅, R₆, R₇, R₈, R₉, R₁₀, R₁₁ et R₁₂ sont choisis parmi un atome d'hydrogène ; un radical halogéno ; un radical alkyle en C₁-C₆ ; un radical styrylalkyle en C₁-C₆ ; un radical cycloalkyle en C₆-C₁₂ ; un radical aryle en C₆-C₁₈ ; un radical amino ; un radical mono ou dialkyl(C₁-C₉)amino ; un radical hydroxyle ; un radical alcoxy en C₁-C₆ ; un radical alcoxy en C₁-C₆ substitué par un radical halogéno, cyano ; un radical aryl(C₆-C₁₈)alcoxy en C₁-C₆ ; un radical mono ou dicycloalkyl(C₆-C₁₂)amino ; un radical mono ou diaryl(C₆-C₁₂)amino ; un radical N,N-(alkyl(C₁-C₉))(aryl(C₆-C₁₈))amino ; un radical N,N-(alkyl(C₁-C₉))(cycloalkyl(C₆-C₁₂))amino ; un radical N,N-(alkyl(C₁-C₉))(aryl(C₆-C₁₈)carbonyl)amino ; un radical alkyl(C₁-C₉)amido ; un radical mono ou di(aryl(C₆-C₁₈)alkyl(C₁-C₉))amino ; un radical aryl(C₆-C₁₈)alcoxy(C₁-C₉) ; un radical alkyl(C₁-C₆)thio ; un radical (aryl(C₆-C₁₈))(alkyl(C₁-C₉))amino dans lequel le radical aryle est substitué par un ou plusieurs radicaux alkyle en C₁-C₆, halogéno ; un radical mono ou diaryl(C₆-C₁₈)amino dans lequel un ou deux radicaux aryle sont substitués par un ou plusieurs radicaux alkyle en C₁-C₆, alcoxy en C₁-C₆, halogéno, trifluorométhyle, alcoxycarbonyle en C₁-C₆, 4-anilinoanilino, anilino, styryle ; un radical mono ou dialkyl(C₁-C₉)amino dans lequel un ou deux radicaux alkyle sont substitués par un ou plusieurs radicaux alcoxy en C₁-C₆, tétrahydrofuryle ; un radical (aryl(C₆-C₁₈)carbonyl)(alkyl(C₁-C₉))amino ; un radical (alkyl(C₁-C₉)carbonyl)(aryl(C₆-C₁₈))amino ; un radical alcénylimino en C₁-C₉ ; un radical aryl(C₆-C₁₈)alcénylimino en C₁-C₉, un groupement monohétérocyclique non aromatique comprenant de 5 à 7 chaînons ;
- R₂ est choisi parmi un atome d'hydrogène ; un radical halogéno ; un radical alkyle en C₁-C₆ ; un radical styrylalkyle en C₁-C₆ ; un radical cycloalkyle en C₆-C₁₂ ; un radical aryle en C₆-C₁₈ ; un radical amino ; un radical alcoxy en C₁-C₆ ; un radical alcoxy en C₁-C₆ substitué par un radical halogéno, cyano ; un radical aryl(C₆-C₁₈)alcoxy en C₁-C₆ ; un radical mono ou dicycloalkyl(C₆-C₁₂)amino ; un radical mono ou diaryl(C₆-C₁₂)amino ; un radical alkyl(C₁-C₉)amido ; un radical mono ou di(aryl(C₆-C₁₈)alkyl(C₁-C₉))amino ; un radical aryl(C₆-C₁₈)alcoxy(C₁-C₉) ; un radical alkyl(C₁-C₆)thio ; un radical mono ou dialkyl(C₁-C₉)amino dans lequel un ou deux radicaux alkyle sont substitués par un ou plusieurs radicaux alcoxy en C₁-C₆, tétrahydrofuryle ; un radical (alkyl(C₁-C₉)carbonyl)(aryl(C₆-C₁₈))amino ; un radical alcénylimino en C₁-C₉ ; un radical aryl(C₆-C₁₈)alcénylimino en C₁-C₉, un groupement monohétérocyclique non aromatique comprenant de 5 à 7 chaînons ;
- deux des radicaux R₁, R₂, R₃, R₄, R₅, R₆, R₇, R₈, R₉, R₁₀, R₁₁ et R₁₂ portés par deux atomes de carbone adjacents pouvant former ensemble un cycle benzène ; un cycle benzène substitué par un radical alcoxy(C₁-C₉)carbonylalkyl(C₁-C₉)amino, α-naphtylalkyl(C₁-C₉)amino.

A titre d'exemple, le composé de formule (I) est tel que :
• R₁, R₃, R₄, R₅, R₆, R₇, R₈, R₉, R₁₀, R₁₁ et R₁₂ sont choisis parmi un atome d'hydrogène ; un radical chloro ; un radical méthyle ; un radical éthyle ; un radical n-propyle ; un radical isopropyle ; un radical n-butyle ; un radical ter-butyle ; un radical cyclohexyle ; un radical phényle ; un radical amino ; un radical méthylamino ; un radical diméthylamino ; un radical éthylamino ; un radical diéthylamino ; un radical n-butylamino ; un radical di(n-butyl)amino ; un radical N,N-(éthyl)(isopentyl)amino ; un radical N,N-(méthyl)(n-propyl)amino ; un radical N,N-(éthyl)(isobutyl)amino ; un radical n-propylamino ; un radical di(n-propyl)amino ; un radical di(n-pentyl)amino ; un radical n-octylamino ; un radical hydroxyle ; un radical méthoxy ; un radical éthoxy ; un radical benzyloxy ; un radical cyclohexylamino ; un radical dibenzylamino ; un radical diphénylamino ; un radical benzylamino ; un radical di(p-tolyl)amino ; un radical anilino ; un radical 2-méthylanilino ; un radical 4-méthoxyanilino ; un radical 2,4-diméthylanilino ; un radical 2,6-diméthylanilino ; un radical 2-chloroanilino ; un radical 2-fluoroanilino ; un radical 3-trifluorométhylanilino ; un radical N-éthyl 4-méthylanilino ; un radical N-éthyl 4-chloroanilino ; un radical N,N-(éthyl)(p-tolyl)amino ; un radical N-méthylanilino ; un radical N-éthyl p-toluidino ; un radical N-méthyl 4-chloroanilino ; un radical N,N-(cyclohexyl)(méthyl)amino ; un radical N,N-(benzoyl)(méthyl)amino ; un radical 2-chloroéthoxy ; un radical 2-cyanoéthoxy ; un radical acétamido ; un radical méthylthio ; un radical 4-(4-anilinoanilino)anilino ; un radical 4-anilinoanilino ; un radical 4-styrylanilino ; un radical N-acétylanilino ; un radical cinnamylamino ; un radical éthylidènamino ; un radical butènylidènamino ; un radical benzylidènamino ; un radical cinnamylidènamino ; un radical N,N-(éthyl)(3-éthoxypropyl)amino ; un radical N,N-(éthyl)(tétrahydrofurfuryl)amino ; un radical pyrrolidino ; un radical pipéridino ; un radical 2-méthoxycarbonylanilino ; un radical éthoxyéthylamino ; un radical N-éthylanilino ; un radical allylamino ; un radical phénéthylamino ;
• R₂ est choisi parmi un atome d'hydrogène un radical chloro ; un radical méthyle ; un radical éthyle ; un radical n-propyle ; un radical isopropyle ; un radical n-butyle ; un radical ter-butyle ; un radical cyclohexyle ; un radical phényle ; un radical amino ; un radical méthoxy ; un radical éthoxy ; un radical benzyloxy ; un radical cyclohexylamino ; un radical dibenzylamino ; un radical diphénylamino ; un radical benzylamino ; un radical anilino ; un radical 2-chloroéthoxy ; un radical 2-cyanoéthoxy ; un radical acétamido ; un radical méthylthio ; un radical N-acétylanilino ; un radical cinnamylamino ; un radical éthylidènamino ; un radical butènylidènamino ; un radical benzylidènamino ; un radical cinnamylidènamino ; un radical pyrrolidino ; un radical pipéridino ; un radical éthoxyéthylamino ; un radical allylamino ; un radical phénéthylamino ;
• deux des radicaux R₁, R₂, R₃, R₄, R₅, R₆, R₇, R₈, R₉, R₁₀, R₁₁ et R₁₂ portés par deux atomes de carbone adjacents forment ensemble un cycle benzène ; un cycle benzène substitué par un radical méthoxycarbonylméthylamino, α-naphtylméthylamino.

Selon un mode de réalisation particulier de l'invention, X est un atome d'oxygène.

A titre d'exemples de composés de formule (I), on peut citer le spiro[isobenzofuran-1,9'-xanthène]-3-one ; le benzo[a]fluorane ; le benzo[c]fluorane ; le 3',6'-diméthoxyfluorane ; le 3'-chloro-6'-diéthylaminofluorane ; le 9'-diéthylaminobenzo[a]fluorane ; le 2'-chloro-6'-diéthylamino-3'-méthylfluorane ; le 3'-chloro-6'-cyclohexylaminofluorane ; le 2'-dibenzylamino-6'-diéthylaminofluorane ; le 2'-anilino-6'-diéthylamino-3'-méthylfluorane ; le 6'-[4-(4-anilinoanilino)anilino]-2'-chloro-3'méthylfluorane ; le 2',7'-dichloro-3',6'-diméthoxyfluorane ; le 3'6'-diéthoxyfluorane ; le 3'6'-dibenzyloxyfluorane ; le 3',6'-bis(2-chloroéthoxy)fluorane ; le 3',6'-bis-(2-cyanoéthoxy)fluorane ; le 6'-amino-2'-t-butylfluorane ; le 6'-amino-2'-t-butyl-3'-méthylfluorane ; le 6'-amino-1,3'-diméthylfluorane ; le 6'-amino-2',3'-diméthylfluorane ; le 3'-amino-6'-chlorofluorane ; le 5'-amino-2',3',7'-triméthylfluorane ; le 2'-chloro-6'-cyclohexylaminofluorane ; le 3'-chloro-6'-cyclohexyl-aminofluorane ; le 6'-cyclohexylamino-2'-méthylfluorane ; le 2'-chloro-6'-méthylaminofluorane ; le 3'-chloro-6'-méthylaminofluorane ; le 3'-chloro-6'-cyclohexyl-amino-4',5'-diméthylfluorane ; le 3'-cyclohexylamino-4,5,6,6',7-pentachlorofluorane ; le 3'-anilino-6'-chlorofluorane ; le 2'-chloro-6'-diéthylaminofluorane ; le 2'-chloro-6'-diéthylamino-3'-méthylfluorane ; le 6'-diéthylamino-2'-méthoxyfluorane ; le 6'-diéthylamino-2'-phénylfluorane ; le 6'-diéthylamino-2'-méthylthiofluorane ; le 2'-chloro-6'-di-n-butylamino-3'-méthylfluorane ; le 3'-chloro-6'-di-n-butylamino-2'-méthylfluorane ; le 2'-chloro-6'-(N-éthyl-4-méthylanilino)fluorane ; le 6'-(N-éthyl-4-méthylanilino)-2'-méthoxyfluorane ; le 6'-(4-chloro-N-éthylanilino)-2',3'-diméthylfluorane ; le 9'-diéthylaminobenzo[a]fluorane ; le 9'-(N-éthyl-N-isopentylamino)benzo[a]fluorane ; le 9'-cyclohexylaminobenzo[a]fluorane ; le 10'-cyclohexyl-benzo[c]fluorane ; le 9'-(2-méthylanilino)benzo[a]fluorane ; le 10'-(4-méthoxyanilino)benzo[c]fluorane ; le 10'-(4-anilinoanilino)benzo[c]fluorane ; le 10'-(4-styrylanilino)benzo[c]fluorane ; le 2'-amino-6'-diéthylaminofluorane ; le 2'-acétamido-6'-diéthylaminofluorane ; le 2'-(N-acétylanilino)-6'-diéthylaminofluorane ; le 2'-(N-benzoyl-N-méthylamino)-6'-diéthylaminofluorane ; le 2'-acétamido-6'-(N-méthylanilino)fluorane ; le 3',6'-bis-(diphénylamino)fluorane ; le 3'-diphénylamino-6'-di-p-tolylaminofluorane ; le 3'-diphénylamino-6'-(N-éthyl-p-toluidino)fluorane ; le 9'-éthylamino-4'-(méthoxycarbonylméthylamino)benzo[a]fluorane ; le 9'-diéthylamino-4'-α-naphthylméthylaminobenzo[a]fluorane ; le 4'-cinnamylamino-10'-diéthylaminobenzo[c]fluorane ; le 2'-n-butylamino-6'-diéthylaminofluorane ; le 6'-diéthylamino-2'-n-octylaminofluorane, 2'-allylamino-6'-diéthylaminofluorane, 6'-diéthylamino-2'-phénéthylaminofluorane; le 2'-benzylamino-6'-diéthylaminofluorane ; le 2'-cinnamylamino-6'-diéthylaminofluorane ; le 2'-dibenzylamino-6'-diéthylamino-3'-méthylfluorane ; le 2'-dibenzylamino-6'-diéthylamino-3'-éthylfluorane ; le 3'-chloro-2'-dibenzylamino-6'-diéthylaminofluorane ; le 6'-(N-éthyl-4-méthylanilino)-3'-méthyl-2'-(N-méthylanilino)fluorane ; le 2'-dibenzylamino-6'-diéthylamino-4'-méthylfluorane ; le 4'-chloro-2'-dibenzylamino-6'-diéthylaminofluorane ; le 2'-dibenzylamino-6'-diéthylamino-4'-méthoxyfluorane ; le 4'-chloro-6'-(N-éthyl-4-méthylanilino)-2'-(N-méthylanilino)fluorane ; le 6'-(N-éthyl-4-méthylanilino)-4'-méthyl-2'-(N-méthylanilino)fluorane ; le 6'-diéthylamino-2'-éthylidènaminofluorane ; le 2'-(2-butènylidènamino)-6'-diéthylaminofluorane ; le 2'-benzylidènamino-6'-diéthylaminofluorane ; le 2'-cinnamylidènamino-6'-diéthylaminofluorane ; le 2'-anilino-6'-diéthylamino-3'-méthylfluorane ; le 2'-anilino-6'-diéthylaminofluorane ; le 2'-anilino-3'-chloro-6'-diéthylaminofluorane ; le 2'-anilino-3'-méthyl-6'-(N-méthyl-N-n-propylamino)fluorane ; le 2'-anilino-6'-(N-cyclohexyl-N-méthylamino)-3'-méthylfluorane ; le 2'-anilino-6'-(N-éthyl-N-isobutylamino)-3'-méthylfluorane ; le 2'-anilino-6'-(N-éthyl-N-isopentylamino)-3'-méthylfluorane ; le 2'-anilino-6'-[N-(3-éthoxypropyl)-N-éthylamino]-3'-méthylfluorane ; le 2'-anilino-6'-(N-éthyl-4-méthylanilino)-3'-méthylfluorane ; le 2'-anilino-6'-(N-éthyl-N-tétrahydrofurfurylamino)-3'-méthylfluorane ; le 2'-anilino-6'-di-n-propylamino-3'-méthylfluorane ; le 2'-anilino-6'-di-n-butylamino-3'-méthylfluorane ; le 2'-anilino-6'-di-n-pentylamino-3'-méthylfluorane ; le 2'-anilino-3'-méthyl-6'-pyrrolidinofluorane ; le 2'-anilino-3'-méthyl-6'-pipéridinofluorane ; le 6'-diéthylamino-2'-(2,4-diméthylanilino)-3'-méthylfluorane ; le 6'-diéthylamino-2'-(2,6-diméthylanilino)-3'-méthylfluorane ; le 2'-(2-chloroanilino)-6'-diéthylaminofluorane ; le 2'-(2-chloroanilino)-6'-di-n-butylaminofluorane ; le 6'-diéthylamino-2'-(2-fluoroanilino)fluorane ; le 6'-di-n-butylamino-2'-(2-fluoroanilino)fluorane ; le 6'-diéthylamino-2'-(3-trifluorométhylanilino)fluorane ; le 6'-diéthylamino-2'-(2-méthoxycarbonylanilino)fluorane ; le 6'-diéthylamino-3'-méthyl-2'-n-octylaminofluorane ; le 3'-chloro-6'-diéthylamino-2'-(2-éthoxyéthylaminofluorane). Le 2'-anilino-6'-diéthylamino-3'-méthylfluorane est particulièrement préféré.

De préférence, lorsque R₂ représente un radical amino, celui-ci est non substitué.

Les composés choisis parmi les composés de formule (I), les colorants correspondant aux composés de formule (I) dont le cycle lactone est ouvert et leurs sels d'addition représentent en général de 0,0001 à 10 % en poids par rapport au poids total de la composition tinctoriale conforme à l'invention.

D'une manière générale, les sels d'addition des composés de formule (I) et des colorants correspondant aux composés de formule (I) dont le cycle lactone est ouvert utilisables dans le cadre de l'invention sont notamment choisis parmi les sels d'addition avec un acide tels que les chlorhydrates, les bromhydrates, les sulfates, les méthosulfates, les gluconates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition conforme à l'invention peut en outre contenir un ou plusieurs colorants directs pouvant notamment être choisis parmi les colorants nitrés de la série benzénique, les colorants directs azoïques, les colorants directs méthiniques et les colorants naturels. Ces colorants directs peuvent être de nature non ionique, anionique ou cationique.

En présence de colorants directs additionnels dans la composition conforme à l'invention, ceux-ci représentent en général de 0,001 à 20 % en poids par rapport au poids total de la composition tinctoriale conforme à l'invention, de préférence de 0,01 à 10 %.

La composition de la présente invention peut en outre comprendre une ou plusieurs colorants d'oxydation choisis parmi les bases d'oxydation et les coupleurs classiquement utilisées en coloration d'oxydation.

En présence de colorants d'oxydation dans la composition conforme à l'invention, ceux-ci représentent en général de 0,001 à 20 % en poids par rapport au poids total de la composition tinctoriale conforme à l'invention, de préférence de 0,01 à 10 %.

Le milieu approprié pour la teinture, appelé aussi support de teinture, est généralement constitué par de l'eau ou par au moins un solvant organique ou par un mélange d'eau et d'au moins un solvant organique. A titre de solvant organique, on peut par exemple citer les cétones tels que l'acétone ; les alcanols en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, et leurs mélanges.

Les composés choisis parmi les composés de formule (I), les colorants correspondant aux composés de formule (I) dont le cycle lactone est ouvert et leurs sels d'addition sont soit solubles, soit en dispersion, dans le support de teinture.

Les solvants sont généralement présents dans des proportions comprises entre 1 et 40 % en poids environ par rapport au poids total de la composition tinctoriale, et encore plus préférentiellement entre 5 et 30 % en poids environ.

La composition conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des polymères cationiques ou amphotères, des cations, des silicones volatiles ou non volatiles, modifiées ou non modifiées, des chitosanes ou des dérivés de chitosane, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Lorsque les solvants sont constitués par de l'eau ou par un mélange d'eau et d'au moins un solvant organique, le pH de la composition tinctoriale conforme à l'invention est généralement compris entre 2 et 12. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, une fonction acide sulfonique, une fonction acide phosphonique ou une fonction acide phosphorique.

Parmi les agents alcalinisants on peut citer, à titre d'exemple :
- les acides aminés basiques ;
- les carbonates ou les bicarbonates de métaux alcalins ou alcalino-terreux ;
- les silicates ou les métasilicates ;
- les composés de formule (II) suivante :

   X(OH)ₙ (II)

   dans laquelle :
   - X représente un ion potassium, lithium, sodium, ammonium N+R₁₃R₁₄R₁₅R₁₆ avec R₁₃, R₁₄, R₁₅ et R₁₆, identiques ou différents, désignant un radical alkyle en C₂-C₄ lorsque n est égal à 1 ;
   - X représente un atome de magnésium ou de calcium lorsque n est égal à 2 ;
- les composés de formule (III) suivante : 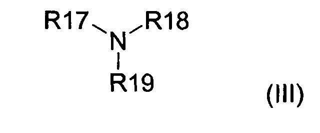 dans laquelle :
   • R₁₇ représente un atome d'hydrogène ; un radical alkyle en C₁-C₆, un radical monohydroxyalkyle en C₁-C₆ ; un radical polyhydroxyalkyle en C₂-C₆ ;
   • R₁₈ et R₁₉, identiques ou différents, représentent un atome d'hydrogène, un radical alkyle en C₁-C₆, un radical monohydroxyalkyle en C₁-C₆ ; un radical polyhydroxyalkyle en C₂-C₆ ;
- les composés de formule (IV) suivante : 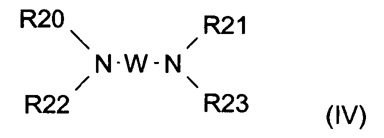 dans laquelle :
   - W est un reste propylène éventuellement substitué par un groupement hydroxyle ou un radical alkyle en C₁-C₄ ;
   - R₂₀, R₂₁, R₂₂ et R₂₃, identiques ou différents, représentent un atome d'hydrogène, un radical alkyle en C₁-C₄ ou hydroxyalkyle en C₁-C₄.

Au sens de la présente invention, on entend par "acide aminé basique", soit (i) un acide aminé présentant en plus de la fonction amine positionnée en α du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22, A. LEHNINGER (1993) pages 115-116, DE BOECK-WESMAEL (1994) pages 57-59.

Les acides aminés basiques conformes à l'invention sont choisis de préférence parmi ceux répondant à la formule (D) suivante : 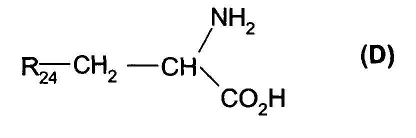 où R₂₄ désigne un groupe choisi parmi :

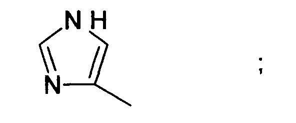

-(CH₂)₃NH₂ ;

-(CH₂)₂NH₂ ;

-(CH₂)₂NHCONH₂ ;

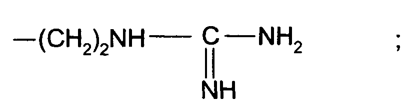

Parmi les composés de formule (D), on peut citer à titre d'exemple l'histidine, la lysine, l'ornithine, la citrulline, l'arginine.

La composition selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de traitement des fibres kératiniques conforme à l'invention permet d'obtenir une coloration intense et tenace dont les reflets peuvent varier en fonction du pH ou de la température et qui peut être effacée et reformée au moins une fois sans perte substantielle de couleur. De préférence, la coloration est modifiée, effacée ou reformée en ajustant le pH à l'aide d'un agent acidifiant ou d'un agent alcalinisant.

Un procédé préféré de traitement des fibres kératiniques, et en particulier des fibres kératiniques humaines telles que les cheveux, caractérisé en ce que les étapes suivantes sont mises en oeuvre :
a) une composition tinctoriale selon l'invention est appliquée sur les fibres kératiniques pendant un temps de pose suffisant, cette application étant éventuellement suivie d'un rinçage, et le pH est ajusté à l'aide d'un agent acidifiant ou d'un agent alcalinisant en fonction de la coloration souhaitée ;
b) éventuellement la coloration des fibres kératiniques est effacée ou modifiée à l'aide d'un agent acidifiant ou d'un agent alcalinisant appliqué sur les fibres kératiniques ;
ou b') éventuellement la coloration des fibres kératiniques est effacée, modifiée ou reformée au moins une fois à l'aide d'un agent acidifiant ou d'un agent alcalinisant appliqué sur les fibres kératiniques.

Selon un mode de réalisation particulier de l'invention, dans la première étape, l'agent acidifiant ou l'agent alcalinisant est mélangée à la composition tinctoriale avant application sur les fibres kératiniques.

Selon un autre mode de réalisation particulier de l'invention, dans la première étape, l'agent acidifiant ou l'agent alcalinisant est appliqué avant ou après la composition tinctoriale.

Dans le sens de la présente invention, la coloration est effacée lorsque les fibres kératiniques ont retrouvé leur couleur d'origine. La coloration est modifiée lorsque la coloration obtenue est différente de celle obtenue au cours de l'étape précédente. La coloration est reformée lorsque la coloration des fibres kératiniques obtenue est la même que celle qui avait été obtenue au cours d'une étape précédente et qui avait ensuite été modifiée.

Le temps de pose de la composition conforme à l'invention est généralement compris entre 1 minute et 1 heure, de préférence entre 15 minutes et 1 heure.

La température d'application est généralement fixée entre la température ambiante et 220°C, de préférence entre la température ambiante et 45 °C.

Les fibres kératiniques peuvent être ou non rincées après application de l'agent acidifiant ou l'agent alcalinisant.

Les agents acidifiants ou alcalinisants sont choisis parmi ceux qui sont décrits ci-dessus.

La présente invention a également pour objet un dispositif à plusieurs compartiments ou kit permettant de mettre en oeuvre le procédé de coloration des fibres kératiniques décrit ci-dessus.

Le dispositif à plusieurs compartiments de l'invention contient dans un premier compartiment une composition conforme à l'invention et dans un deuxième compartiment un agent acidifiant ou un agent alcalinisant. Dans une autre version, le dispositif à plusieurs compartiments de l'invention contient dans le deuxième compartiment un agent acidifiant et dans un troisième compartiment un agent alcalinisant.

La présente invention a également pour objet une composition pour la coloration des fibres kératiniques, et en particulier des fibres kératiniques humaines telles que les cheveux telle que définie ci-dessus comprenant de plus un polymère épaississant associatif ou non associatif.

On peut citer à titre d'exemple de polymère épaississant associatif les dérivés de polyuréthanes associatifs, les dérivés de celluloses associatifs, les dérivés de polyvinyllactames associatifs et les dérivés de polyacides insaturés associatifs.

On peut citer à titre d'exemple de polymère épaississant non associatif les homopolymères d'acide acrylique réticulés ; les homopolymères réticulés d'acide 2-acrylamido-2-méthyl-propane sulfonique et leurs copolymères réticulés d'acrylamide partiellement ou totalement neutralisés ; les homopolymères d'acrylate d'ammonium ou les copolymères d'acrylate d'ammonium et d'acrylamide ; les homopolymères de diméthylaminoéthylméthacrylate quaternisé par le chlorure de méthyle ou les copolymères de diméthylamino-éthylméthacrylate quaternisé par le chlorure de méthyle et d'acrylamide ; les gommes de guar non ioniques ; les gommes de biopolysaccharides d'origine microbienne telles que les gommes de scléroglucane ou de xanthane ; les gommes issues d'exudats végétaux telles que les gommes arabiques, gommes Ghatti, gommes Karaya et Tragacanthe ; les hydroxypropyl ou carboxyméthyl celluloses; les pectines ; les alginates.

L'exemple qui suit sert à illustrer l'invention sans toutefois présenter un caractère limitatif.

### EXEMPLE

La composition tinctoriale ci-dessous est préparée :

| | composition |
|---|---|
| 3-diéthylamino 6-méthyl 7-anilino fluorane | 2 g |
| Acétone | q.s.p. 100 g |

La composition est appliquée sur des mèches de cheveux gris à 90 % de cheveux blancs naturels et permanentés, à raison de 10 g pour 1 g de cheveux, à température ambiante pendant une minute. La couleur est ensuite révélée par application d'une solution d'acide sulfurique 1 M sur les mèches. A l'issue de la révélation de la couleur, la mèche est séchée.

La coloration capillaire est évaluée de manière visuelle. La nuance obtenue est un vert.

Les mèches ainsi colorées peuvent être décolorées très rapidement par application d'une solution de soude 0,1 M. Les mèches retrouvent leurs reflets d'origine.

## Revendications

1. Utilisation pour la coloration des fibres kératiniques, et en particulier des fibres kératiniques humaines telles que les cheveux, d'une composition comprenant, dans un milieu approprié pour la teinture, au moins un composé choisi parmi les composés comportant un cycle lactone de formule (I), les colorants correspondant aux composés de formule (I) dont le cycle lactone est ouvert et leurs sels d'addition : 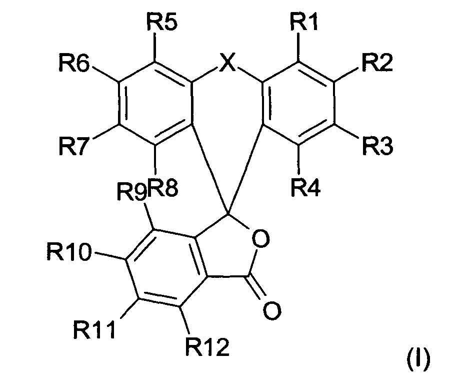 dans laquelle :
• R₁, R₃, R₄, R₅, R₆, R₇, R₈, R₉, R₁₀, R₁₁ et R₁₂ représentent, indépendamment l'un de l'autre :
- un atome d'hydrogène ;
- un radical halogéno ;
- un radical hydroxyle ;
- un radical nitro ;
- un radical amino ;
- un radical carboxy ;
- un radical aminocarbonyle ;
- un radical cyano ;
- un radical issu d'une chaîne hydrocarbonée comportant de 1 à 100 atomes de carbone, de préférence 1 à 50, linéaire ou ramifiée, acyclique ou mono ou polycyclique, condensée ou non condensée, saturée ou insaturée, aromatique ou non aromatique, pouvant être interrompue par un ou plusieurs hétéroatomes choisis parmi les atomes d'azote, d'oxygène et de soufre ou par un ou plusieurs groupements carbonyle, pouvant être terminée par un groupement hydrogénocarbonyle ou par un groupement contenant un ou plusieurs hétéroatomes choisis parmi les atomes d'azote, d'oxygène et de soufre et pouvant être substituée par un ou plusieurs groupements choisis parmi les radicaux hydroxyle, halogéno, carboxy, carboxyalkyle en C₁-C₉, cyano, amino ;
les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₆-C₁₂ ; un radical aryle en C₆-C₁₈ ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ;
• R₂ représente :
- un atome d'hydrogène ;
- un radical halogéno ;
- un radical nitro ;
- un radical amino ;
- un radical carboxy ;
- un radical aminocarbonyle ;
- un radical cyano ;
- un radical issu d'une chaîne hydrocarbonée comportant de 1 à 100 atomes de carbone, de préférence 1 à 50, linéaire ou ramifiée, acyclique ou mono ou polycyclique; condensée ou non condensée, saturée ou insaturée, aromatique ou non aromatique, pouvant être interrompue par un ou plusieurs hétéroatomes choisis parmi les atomes d'azote, d'oxygène et de soufre ou par un ou plusieurs groupements carbonyle, pouvant être terminée par un groupement hydrogénocarbonyle ou par un groupement contenant un ou plusieurs hétéroatomes choisis parmi les atomes d'azote, d'oxygène et de soufre et pouvant être substituée par un ou plusieurs groupements choisis parmi les radicaux hydroxyle, halogéno, carboxy, carboxyalkyle en C₁-C₉, cyano, amino ;
les radicaux amino étant non substitués ou substitués par un ou deux radicaux, identiques ou différents, choisis parmi un radical alkyle en C₁-C₉ substitué ; un radical hydroxyalkyle en C₁-C₉ ; un radical alcényle en C₂-C₉ ; un radical cycloalkyle en C₆-C₁₂ ; un radical aryle en C₆-C₁₈ non substitué ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ;
• deux des radicaux R₁, R₂, R₃, R₄, R₅, R₆, R₇, R₈, R₉, R₁₀, R₁₁ et R₁₂ portés par deux atomes de carbone adjacents pouvant former ensemble un groupement mono ou polycarbocyclique aromatique, condensé ou non condensé, comprenant de 5 à 20 chaînons, un ou plusieurs atomes de carbone pouvant être remplacés par un atome d'oxygène, d'azote, de soufre ou de phosphore, non substitué ou substitué par un ou plusieurs atomes d'halogène, un ou plusieurs radicaux hydroxyle, amino, carboxy, aryle en C₆-C₁₈, cyano ;
les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₆-C₁₂ ; un radical aryle en C₆-C₁₈ ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ;
• X représente un atome de carbone, d'azote, de soufre, d'oxygène, de phosphore.

2. Utilisation selon la revendication 1 dans laquelle le composé de formule (I) est tel que :
• R₁, R₃, R₄, R₅, R₆, R₇, R₈, R₉, R₁₀, R₁₁ et R₁₂ représentent, indépendamment l'un de l'autre :
- un atome d'hydrogène ;
- un radical halogéno ;
- un radical hydroxyle ;
- un radical alkyle en C₁-C₉ ;
- un radical cycloalkyle en C₆-C₁₂ ;
- un radical aryle en C₆-C₁₈ ;
- un radical alcoxy en C₁-C₉ ;
- un radical alkylthio en C₁-C₉ ;
- un radical amino ;
- un radical mono ou dialkyl(C₁-C₉)amino ;
- un radical mono ou dicycloalkyl(C₆-C₁₂)amino ;
- un radical mono ou diaryl(C₆-C₁₈)amino ;
- un radical mono ou di(aryl(C₆-C₁₈)alkyl(C₁-C₉))amino ;
- un radical N,N-(cycloalkyl(C₆-C₁₂))(alkyl(C₁-C₉))amino ;
- un radical N,N-(aryl(C₆-C₁₈))(alkyl(C₁-C₉))amino ;
- un radical N,N-(aryl(C₆-C₁₈)carbonyl)(alkyl(C₁-C₉))amino ;
- un radical N,N-(alkyl(C₁-C₉)carbonyl)(aryl(C₈-C₁₈))amino ;
- un radical alkyl(C₁-C₆)carbonylalkyl(C₁-C₆) ;
- un radical alkyle en C₁-C₆ substitué par un groupement mono ou polyhétérocyclique, condensé ou non condensé, aromatique ou non aromatique, comprenant de 5 à 50 chaînons ;
- un groupement mono ou polyhétérocyclique, condensé ou non condensé, aromatique ou non aromatique, comprenant de 5 à 50 chaînons ;
les groupements hétérocycliques pouvant être substitués par un ou plusieurs radicaux hydroxyle, halogéno, amino, alcoxy en C₁-C₉ ;
- un radical aryl(C₆-C₁₈)alcoxy(C₁-C₉) ;
- un radical (alkyl(C₁-C₉)carbonyl)amino ;
- un radical aminoaryl(C₆-C₁₈) amido ;
- un radical aminoalcényle en C₂-C₉ ;
- un radical aryl(C₆-C₁₈)imino ;
- un radical ary)(C₆-C₁₈)alcényl(C₂-C₉)imino ;
- un radical alcényl(C₂-C₉)imino ;
- un radical hydrogénocarbonylalcoxy(C₁-C₉) ;
les groupements alkyle ou alcényle étant non substitués ou substitués par un ou plusieurs groupements hydroxyle, halogéno, amino, cyano, thio, alcoxy en C₁-C₉, styryle, tétrahydrofuryle, carboxy ou alcoxycarbonyle en C₁-C₉ et les groupements cycloalkyle ou aryle étant non substitués ou substitués par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle ;
• R₂ représente :
- un atome d'hydrogène ;
- un radical halogéno ;
- un radical alkyle en C₁-C₉ ;
- un radical cycloalkyle en C₆-C₁₂ ;
- un radical aryle en C₆-C₁₈ ;
- un radical alcoxy en C₁-C₉ ;
- un radical alkylthio en C₁-C₉ ;
- un radical amino ;
- un radical mono ou dicycloalkyl(C₆-C₁₂)amino ;
- un radical mono ou di(aryl(C₆-C₁₈)alkyl(C₁-C₉))amino ;
- un radical alkyl(C₁-C₆)carbonylalkyl(C₁-C₆) ;
- un radical alkyle en C₁-C₆ substitué par un groupement mono ou polyhétérocyclique, condensé ou non condensé, aromatique ou non aromatique, comprenant de 5 à 50 chaînons ;
- un groupement mono ou polyhétérocyclique, condensé ou non condensé, aromatique ou non aromatique, comprenant de 5 à 50 chaînons ;
les groupements hétérocycliques pouvant être substitués par un ou plusieurs radicaux hydroxyle, halogéno, amino, alcoxy en C₁-C₉ ;
- un radical aryl(C₆-C₁₈)alcoxy(C₁-C₉) ;
- un radical (alkyl(C₁-C₉)carbonyl)amino ;
- un radical aminoaryl(C₆-C₁₈) amido ;
- un radical aminoalcényle en C₂-C₉ ;
- un radical aryl(C₆-C₁₈)imino ;
- un radical aryl(C₆-C₁₈)alcényl(C₂-C₉)imino ;
- un radical alcényl(C₂-C₉)imino ;
- un radical hydrogénocarbonylalcoxy(C₁-C₉) ;
les groupements alkyle ou alcényle étant non substitués ou substitués par un ou plusieurs groupements hydroxyle, halogéno, amino, cyano, thio, alcoxy en C₁-C₉, styryle, tétrahydrofuryle, carboxy ou alcoxycarbonyle en C₁-C₉ et les groupements cycloalkyle ou aryle étant non substitués ou substitués par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle
- un radical N,N-(aryl(C₆-C₁₈)carbonyl)(alkyl(C₁-C₉))amino ;
le groupement alkyle étant substitué par un ou plusieurs groupements hydroxyle, halogéno, amino, cyano, thio, alcoxy en C₁-C₉, styryle, tétrahydrofuryle, carboxy ou alcoxycarbonyle en C₁-C₉ et le groupement aryle étant non substitué ou substitué par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-Cg, anilino, 4-anilinoanilino, styryle ;
- un radical N,N-(aryl(C₆-C₁₈))(alkyl(C₁-C₉))amino ;
- un radical mono ou dialkyl(C₁-C₉)amino ;
- un radical mono ou diaryl(C₆-C₁₈)amino ;
- un radical N,N-(cycloalkyl(C₆-C₁₂))(alkyl(C₁-C₉))amino ;
les groupements alkyle étant substitués par un ou plusieurs groupements hydroxyle, halogéno, amino, cyano, thio, alcoxy en C₁-C₉, styryle, tétrahydrofuryle, carboxy ou alcoxycarbonyle en C₁-C₉ ; le groupement cycloalkyle étant non substitué ou substitué par un ou plusieurs radicaux hydroxyle, halogéno, amino, alkyle en C₁-C₉, alcoxy en C₁-C₉, mono ou diaminoaryl(C₆-C₁₈)alkyl(C₁-C₉), trifluorométhyle, alcoxycarbonyle en C₁-C₉, anilino, 4-anilinoanilino, styryle ; les groupements aryle étant non substitués ;
- un radical N,N-(alkyl(C₁-C₉)carbonyl)(aryl)(C₆-C₁₈))amino ;
le groupement alkyle étant non substitué ou substitué par un ou plusieurs groupements hydroxyle, halogéno, amino, cyano, thio, alcoxy en C₁-C₉, styryle, tétrahydrofuryle, carboxy ou alcoxycarbonyle en C₁-C₉ et le groupement aryle étant non substitué ;
• deux des radicaux R₁, R₂, R₃, R₄, R₅, R₆, R₇, R₈, R₉, R₁₀, R₁₁ et R₁₂ portés par deux atomes de carbone adjacents pouvant former ensemble un groupement mono ou polycarbocyclique aromatique, condensé ou non condensé, comprenant de 5 à 20 chaînons, un ou plusieurs atomes de carbone pouvant être remplacés par un atome d'oxygène, d'azote, de soufre ou de phosphore, non substitué ou substitué par un ou plusieurs atomes d'halogène, un ou plusieurs radicaux hydroxyle, amino, mono ou dialkyl(C₁-C₄)amino, carboxy, alcoxy(C₁-C₉)carbonylalkyl(C₁-C₉)amino, α-naphtylalkylamino ;
• X représente un atome de carbone, d'azote, de soufre, d'oxygène, de phosphore.

3. Utilisation selon la revendication 1 ou 2, dans laquelle le composé de formule (I) est tel que :
• R₁, R₃, R₄, R₅, R₆, R₇, R₈, R₉, R₁₀, R₁₁ et R₁₂ sont choisis parmi un atome d'hydrogène ; un radical halogéno ; un radical alkyle en C₁-C₆ ; un radical styrylalkyle en C₁-C₆ ; un radical cycloalkyle en C₆-C₁₂ ; un radical aryle en C₆-C₁₈ ; un radical amino ; un radical mono ou dialkyl(C₁-C₉)amino ; un radical hydroxyle ; un radical alcoxy en C₁-C₆ ; un radical alcoxy en C₁-C₆ substitué par un radical halogéno, cyano ; un radical aryl(C₆-C₁₈)alcoxy en C₁-C₆ ; un radical mono ou dicycloalkyl(C₆-C₁₂)amino ; un radical mono ou diaryl(C₆-C₁₂)amino ; un radical N,N-(alkyl(C₁-C₉))(aryl(C₆-C₁₈))amino ; un radical N,N-(alkyl(C₁-C₉))(cycloalkyl(C₆-C₁₂))amino ; un radical N,N-(alkyl(C₁-C₉))(aryl(C₆-C₁₈)carbonyl)amino ; un radical alkyl(C₁-C₉)amido ; un radical mono ou di(aryl(C₆-C₁₈)alkyl(C₁-C₉))amino ; un radical aryl(C₆-C₁₈)alcoxy(C₁-C₉) ; un radical alkyl(C₁-C₆)thio ; un radical (aryl(C₆-C₁₈))(alkyl(C₁-C₉))amino dans lequel le radical aryle est substitué par un ou plusieurs radicaux alkyle en C₁-C₆, halogéno ; un radical mono ou diaryl(C₆-C₁₈)amino dans lequel un ou deux radicaux aryle sont substitués par un ou plusieurs radicaux alkyle en C₁-C₆, alcoxy en C₁-C₆, halogéno, trifluorométhyle, alcoxycarbonyle en C₁-C₆, 4-anilinoanilino, anilino, styryle ; un radical mono ou dialkyl(C₁-C₉)amino dans lequel un ou deux radicaux alkyle sont substitués par un ou plusieurs radicaux alcoxy en C₁-C₆, tétrahydrofuryle ; un radical (aryl(C₆-C₁₈)carbonyl)(alkyl(C₁-C₉)amino ; un radical (alkyl(C₁-C₉)carbonyl)(aryl(C₆-C₁₈))amino ; un radical alcénylimino en C₁-C₉ ; un radical aryl(C₆-C₁₈)alcénylimino en C₁-C₉, un groupement monohétérocyclique non aromatique comprenant de 5 à 7 chaînons ;
• R₂ est choisi parmi un atome d'hydrogène ; un radical halogéno ; un radical alkyle en C₁-C₆ ; un radical styrylalkyle en C₁-C₆ ; un radical cycloalkyle en C₆-C₁₂ ; un radical aryle en C₆-C₁₈ ; un radical amino ; un radical alcoxy en C₁-C₆ ; un radical alcoxy en C₁-C₆ substitué par un radical halogéno, cyano ; un radical aryl(C₆-C₁₈)alcoxy en C₁-C₆ ; un radical mono ou dicycloalkyl(C₆-C₁₂)amino ; un radical mono ou diaryl(C₆-C₁₂)amino ; un radical alkyl(C₁-C₉)amido ; un radical mono ou di(aryl(C₆-C₁₈)alkyl(C₁-C₉))amino ; un radical aryl(C₆-C₁₈)alcoxy(C₁-C₉) ; un radical alkyl(C₁-C₆)thio ; un radical mono ou dialkyl(C₁-C₉)amino dans lequel un ou deux radicaux alkyle sont substitués par un ou plusieurs radicaux alcoxy en C₁-C₆, tétrahydrofuryle ; un radical (alkyl(C₁-C₉)carbonyl)(aryl(C₆-C₁₈))amino ; un radical alcénylimino en C₁-C₉ ; un radical aryl(C₆-C₁₈)alcénylimino en C₁-C₉, un groupement monohétérocyclique non aromatique comprenant de 5 à 7 chaînons ;
• deux des radicaux R₁, R₂, R₃, R₄, R₅, R₆, R₇, R₈, R₉, R₁₀, R₁₁ et R₁₂ portés par deux atomes de carbone adjacents pouvant former ensemble un cycle benzène ; un cycle benzène substitué par un radical alcoxy(C₁-C₉)carbonylalkyl(C₁-C₉)amino, α-naphtylalkyl(C₁-C₉)amino.

4. Utilisation selon la revendication 3, dans laquelle le composé de formule (I) est tel que :
• R₁, R₃, R₄, R₅, R₆, R₇, R₈, R₉, R₁₀, R₁₁ et R₁₂ sont choisis parmi un atome d'hydrogène ; un radical chloro ; un radical méthyle ; un radical éthyle ; un radical n-propyle ; un radical isopropyle ; un radical n-butyle ; un radical ter-butyle ; un radical cyclohexyle ; un radical phényle ; un radical amino ; un radical méthylamino ; un radical diméthylamino ; un radical éthylamino ; un radical diéthylamino ; un radical n-butylamino ; un radical di(n-butyl)amino ; un radical N,N-(éthyl)(isopentyl)amino ; un radical N,N-(méthyl)(n-propyl)amino ; un radical N,N-(éthyl)(isobutyl)amino ; un radical n-propylamino ; un radical di(n-propyl)amino ; un radical di(n-pentyl)amino ; un radical n-octylamino ; un radical hydroxyle ; un radical méthoxy ; un radical éthoxy ; un radical benzyloxy ; un radical cyclohexylamino ; un radical dibenzylamino ; un radical diphénylamino ; un radical benzylamino ; un radical di(p-tolyl)amino ; un radical anilino ; un radical 2-méthylanilino ; un radical 4-méthoxyanilino ; un radical 2,4-diméthylanilino ; un radical 2,6-diméthylanilino ; un radical 2-chloroanilino ; un radical 2-fluoroanilino ; un radical 3-trifluorométhylanilino ; un radical N-éthyl 4-méthylanilino ; un radical N-éthyl 4-chloroanilino ; un radical N,N-(éthyl)(p-tolyl)amino ; un radical N-méthylanilino ; un radical N-éthyl p-toluidino ; un radical N-méthyl 4-chloroanilino ; un radical N,N-(cyclohexyl)(méthyl)amino ; un radical N,N-(benzoyl)(méthyl)amino ; un radical 2-chloroéthoxy ; un radical 2-cyanoéthoxy ; un radical acétamido ; un radical méthylthio ; un radical 4-(4-anilinoanilino)anilino ; un radical 4-anilinoanilino ; un radical 4-styrylanilino ; un radical N-acétylanilino ; un radical cinnamylamino ; un radical éthylidènamino ; un radical butènylidènamino ; un radical benzylidènamino ; un radical cinnamylidènamino ; un radical N,N-(éthyl)(3-éthoxypropyl)amino ; un radical N,N-(éthyl)(tétrahydrofurfuryl)amino ; un radical pyrrolidino ; un radical pipéridino ; un radical 2-méthoxycarbonylanilino ; un radical éthoxyéthylamino ; un radical N-éthylanilino ; un radical allylamino ; un radical phénéthylamino ;
• R₂ est choisi parmi un atome d'hydrogène ; un radical chloro ; un radical méthyle ; un radical éthyle ; un radical n-propyle ; un radical isopropyle ; un radical n-butyle ; un radical ter-butyle ; un radical cyclohexyle ; un radical phényle ; un radical amino ; un radical méthoxy ; un radical éthoxy ; un radical benzyloxy ; un radical cyclohexylamino ; un radical dibenzylamino ; un radical diphénylamino ; un radical benzylamino ; un radical anilino ; un radical 2-chloroéthoxy ; un radical 2-cyanoéthoxy ; un radical acétamido ; un radical méthylthio ; un radical N-acétylanilino ; un radical cinnamylamino ; un radical éthylidènamino ; un radical butènylidènamino ; un radical benzylidènamino ; un radical cinnamylidènamino ; un radical pyrrolidino ; un radical pipéridino ; un radical éthoxyéthylamino ; un radical allylamino ; un radical phénéthylamino ;
• deux des radicaux R₁, R₂, R₃, R₄, R₅, R₆, R₇, R₈, R₉, R₁₀, R₁₁ et R₁₂ portés par deux atomes de carbone adjacents forment ensemble un cycle benzène ; un cycle benzène substitué par un radical méthoxycarbonylméthylamino, α-naphtylméthylamino.

5. Utilisation selon l'une quelconque des revendications précédentes, dans laquelle le composé de formule (I) est tel que X est un atome d'oxygène.

6. Utilisation selon l'une quelconque des revendications précédentes, dans laquelle le composé de formule (I) est choisi parmi le spiro[isobenzofuran-1,9'-xanthène]-3-one ; le benzo[a]fluorane ; le benzo[c]fluorane ; le 3',6'-diméthoxyfluorane ; le 3'-chloro-6'-diéthylaminofluorane ; le 9'-diéthylaminobenzo[a]fluorane ; le 2'-chloro-6'-diéthylamino-3'-méthylfluorane ; le 3'-chloro-6'-cyclohexylaminofluorane ; le 2'-dibenzylamino-6'-diéthylaminofluorane ; le 2'-anilino-6'-diéthylamino-3'-méthylfluorane ; le 6'-[4-(4-anilinoanilino)anilino]-2'-chloro-3'méthylfluorane ; le 2',7'-dichloro-3',6'-diméthoxyfluorane ; le 3'6'-diéthoxyfluorane ; le 3'6'-dibenzyloxyfluorane ; le 3',6'-bis(2-chloroéthoxy)fluorane ; le 3',6'-bis-(2-cyanoéthoxy)fluorane ; le 6'-amino-2'-t-butylfluorane ; le 6'-amino-2'-t-butyl-3'-méthylfluorane ; le 6'-amino-1,3'-diméthylfluorane ; le 6'-amino-2',3'-diméthylfluorane ; le 3'-amino-6'-chlorofluorane ; le 5'-amino-2',3',7'-triméthylfluorane ; le 2'-chloro-6'-cyclohexylaminofluorane ; le 3'-chloro-6'-cyclohexyl-aminofluorane ; le 6'-cyclohexylamino-2'-méthylfluorane ; le 2'-chloro-6'-méthylaminofluorane ; le 3'-chloro-6'-méthylaminofluorane ; le 3'-chloro-6'-cyclohexyl-amino-4',5'-diméthylfluorane ; le 3'-cyclohexylamino-4,5,6,6',7-pentachlorofluorane ; le 3'-anilino-6'-chlorofluorane ; le 2'-chloro-6'-diéthylaminofluorane ; le 2'-chloro-6'-diéthylamino-3'-méthylfluorane ; le 6'-diéthylamino-2'-méthoxyfluorane ; le 6'-diéthylamino-2'-phénylfluorane ; le 6'-diéthylamino-2'-méthylthiofluorane ; le 2'-chloro-6'-di-n-butylamino-3'-méthylfluorane ; le 3'-chloro-6'-di-n-butylamino-2'-méthylfluorane ; le 2'-chloro-6'-(N-éthyl-4-méthylanilino)fluorane ; le 6'-(N-éthyl-4-méthylanilino)-2'-méthoxyfluorane ; le 6'-(4-chloro-N-éthylanilino)-2',3'-diméthylfluorane ; le 9'-diéthylaminobenzo[a]fluorane ; le 9'-(N-éthyl-N-isopentylamino)benzo[a]fluorane ; le 9'-cyclohexylaminobenzo[a]fluorane ; le 10'-cyclohexyl-benzo[c]fluorane ; le 9'-(2-méthylanilino)benzo[a]fluorane ; le 10'-(4-méthoxyanilino)benzo[c]fluorane ; le 10'-(4-anilinoanilino)benzo[c]fluorane ; le 10'-(4-styrylanilino)benzo[c]fluorane ; le 2'-amino-6'-diéthylaminofluorane ; le 2'-acétamido-6'-diéthylaminofluorane ; le 2'-(N-acétylanilino)-6'-diéthylaminofluorane ; le 2'-(N-benzoyl-N-méthylamino)-6'-diéthylaminofluorane ; le 2'-acétamido-6'-(N-méthylanilino)fluorane ; le 3',6'-bis-(diphénylamino)fluorane ; le 3'-diphénylamino-6'-di-p-tolylaminofluorane ; le 3'-diphénylamino-6'-(N-éthyl-p-toluidino)fluorane ; le 9'-éthylamino-4'-(méthoxycarbonylméthylamino)benzo[a]fluorane ; le 9'-diéthylamino-4'-α-naphthylméthylaminobenzo[a]fluorane ; le 4'-cinnamylamino-10'-diéthylaminobenzo[c]fluorane ; le 2'-n-butylamino-6'-diéthylaminofluorane ; le 6'-diéthylamino-2'-n-octylaminofluorane, 2'-allylamino-6'-diéthylaminofluorane, 6'-diéthylamino-2'-phénéthylaminofluorane ; le 2'-benzylamino-6'-diéthylaminofluorane ; le 2'-cinnamylamino-6'-diéthylaminofluorane ; le 2'-dibenzylamino-6'-diéthylamino-3'-méthylfluorane ; le 2'-dibenzylamino-6'-diéthylamino-3'-éthylfluorane ; le 3'-chloro-2'-dibenzylamino-6'-diéthylaminofluorane ; le 6'-(N-éthyl-4-méthylanilino)-3'-méthyl-2'-(N-méthylanilino)fluorane ; le 2'-dibenzylamino-6'-diéthylamino-4'-méthylfluorane ; le 4'-chloro-2'-dibenzylamino-6'-diéthylaminofluorane ; le 2'-dibenzylamino-6'-diéthylamino-4'-méthoxyfluorane ; le 4'-chloro-6'-(N-éthyl-4-méthylanilino)-2'-(N-méthylanilino)fluorane ; le 6'-(N-éthyl-4-méthylanilino)-4'-méthyl-2'-(N-méthylanilino)fluorane ; le 6'-diéthylamino-2'-éthylidènaminofluorane ; le 2'-(2-butènylidènamino)-6'-diéthylaminofluorane ; le 2'-benzylidènamino-6'-diéthylaminofluorane ; le 2'-cinnamylidènamino-6'-diéthylaminofluorane ; le 2'-anilino-6'-diéthylamino-3'-méthylfluorane ; le 2'-anilino-6'-diéthylaminofluorane ; le 2'-anilino-3'-chloro-6'-diéthylaminofluorane ; le 2'-anilino-3'-méthyl-6'-(N-méthyl-N-n-propylamino)fluorane ; le 2'-anilino-6'-(N-cyclohexyl-N-méthylamino)-3'-méthylfluorane ; le 2'-anilino-6'-(N-éthyl-N-isobutylamino)-3'-méthylfluorane ; le 2'-anilino-6'-(N-éthyl-N-isopentylamino)-3'-méthylfluorane ; le 2'-anilino-6'-[N-(3-éthoxypropyl)-N-éthylamino]-3'-méthylfluorane ; le 2'-anilino-6'-(N-éthyl-4-méthylanilino)-3'-méthylfluorane ; le 2'-anilino-6'-(N-éthyl-N-tétrahydrofurfurylamino)-3'-méthylfluorane ; le 2'-anilino-6'-di-n-propylamino-3'-méthylfluorane ; le 2'-anilino-6'-di-n-butylamino-3'-méthylfluorane ; le 2'-anilino-6'-di-n-pentylamino-3'-méthylfluorane ; le 2'-anilino-3'-méthyl-6'-pyrrolidinofluorane ; le 2'-anilino-3'-méthyl-6'-pipéridinofluorane ; le 6'-diéthylamino-2'-(2,4-diméthylanilino)-3'-méthylfluorane ; le 6'-diéthylamino-2'-(2,6-diméthylanilino)-3'-méthylfluorane ; le 2'-(2-chloroanilino)-6'-diéthylaminofluorane ; le 2'-(2-chloroanilino)-6'-di-n-butylaminofluorane ; le 6'-diéthylamino-2'-(2-fluoroanilino)fluorane ; le 6'-di-n-butylamino-2'-(2-fluoroanilino)fluorane ; le 6'-diéthylamino-2'-(3-trifluorométhylanilino)fluorane ; le 6'-diéthylamino-2'-(2-méthoxycarbonylanilino)fluorane ; le 6'-diéthylamino-3'-méthyl-2'-n-octylaminofluorane ; le 3'-chloro-6'-diéthylamino-2'-(2-éthoxyéthylaminofluorane).

7. Utilisation selon l'une quelconque des revendications précédentes, dans laquelle lorsque R₂ représente un radical amino, celui-ci est non substitué.

8. Utilisation selon l'une quelconque des revendications précédentes, dans laquelle les composés choisis parmi les composés de formule (I), les colorants correspondant aux composés de formule (I) dont le cycle lactone est ouvert et leurs sels d'addition représentent de 0,0001 à 10 % en poids, rapporté au poids total de la composition.

9. Utilisation selon l'une quelconque des revendications précédentes, dans laquelle le milieu approprié pour la teinture est constitué par de l'eau ou par au moins un solvant organique ou par un mélange d'eau et d'au moins un solvant organique.

10. Utilisation selon la revendication 9, dans laquelle le milieu approprié pour la teinture est constitué par de l'eau ou par un mélange d'eau et d'au moins un solvant organique et le pH est compris entre 2 et 12.

11. Procédé de traitement des fibres kératiniques, et en particulier des fibres kératiniques humaines telles que les cheveux, **caractérisé en ce que** les étapes suivantes sont mises en oeuvre :
a) une composition tinctoriale telle que définie à l'une quelconque des revendications 1 à 10 est appliquée sur les fibres kératiniques pendant un temps de pose suffisant, cette application étant éventuellement suivie d'un rinçage, et le pH est ajusté à l'aide d'un agent acidifiant ou d'un agent alcalinisant en fonction de la coloration souhaitée ;
b) éventuellement la coloration des fibres kératiniques est effacée ou modifiée à l'aide d'un agent acidifiant ou d'un agent alcalinisant appliqué sur les fibres kératiniques ;
ou b') éventuellement la coloration des fibres kératiniques est effacée, modifiée ou reformée au moins une fois à l'aide d'un agent acidifiant ou d'un agent alcalinisant appliqué sur les fibres kératiniques.

12. Procédé selon la revendication 11, dans lequel, dans la première étape, l'agent acidifiant ou l'agent alcalinisant est mélangée à la composition tinctoriale avant application sur les fibres kératiniques.

13. Procédé selon la revendication 11, dans lequel, dans la première étape, l'agent acidifiant ou l'agent alcalinisant est appliqué avant ou après la composition tinctoriale.

14. Procédé selon l'une quelconque des revendications 11 à 13, dans lequel les fibres kératiniques sont rincées après application de l'agent acidifiant ou l'agent alcalinisant.

15. Procédé selon l'une quelconque des revendications 11 à 14, dans lequel l'agent acidifiant est choisi parmi les acides minéraux tels que l'acide chlorhydrique, l'acide nitrique, l'acide sulfurique ou les acides organiques tels que les composés comprenant au moins une fonction acide carboxylique, une fonction acide sulfonique, une fonction acide phosphonique ou une fonction acide phosphorique.

16. Procédé selon l'une quelconque des revendications 11 à 15, dans lequel l'agent alcalinisant est choisi parmi :
- les acides aminés basiques ;
- les carbonates ou les bicarbonates de métaux alcalins ou alcalino-terreux ;
- les silicates ou les métasilicates ;
- les composés de formule (II) suivante :
X(OH)ₙ (II)
dans laquelle :
• X représente un ion potassium, lithium, sodium, ammonium N⁺R₁₃R₁₄R₁₅R₁₆ avec R₁₃, R₁₄, R₁₅ et R₁₆, identiques ou différents, désignant un radical alkyle en C₂-C₄ lorsque n est égal à 1 ;
• X représente un atome de magnésium ou de calcium lorsque n est égal à 2 ;
- les composés de formule (III) suivante : 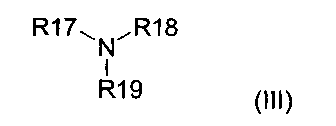 dans laquelle:
• R₁₇ représente un atome d'hydrogène ; une radical alkyle en C₁-C₆, une radical monohydroxyalkyle en C₁-C₆ ; une radical polyhydroxyalkyle en C₂-C₆ ;
• R₁₈ et R₁₉, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ;
- les composés de formule (IV) suivante : 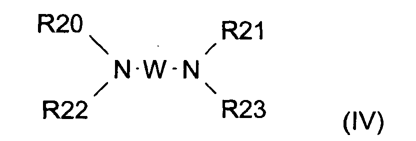 dans laquelle :
• W est un reste propylène éventuellement substitué par un groupement hydroxyle ou un radical alkyle en C₁-C₄ ;
• R₂₀. R₂₁, R₂₂ et R₂₃, identiques ou différents, représentent un atome d'hydrogène, un radical alkyle en C₁-C₄ ou hydroxyalkyle en C₁-C₄.

17. Dispositif à plusieurs compartiments contenant dans un premier compartiment une composition telle que définie à l'une quelconque des revendications 1 à 10 et dans un deuxième compartiment un agent acidifiant ou un agent alcalinisant.

18. Dispositif selon la revendication 17, contenant dans le deuxième compartiment un agent acidifiant et dans un troisième compartiment un agent alcalinisant.

19. Composition telle que définie à l'une quelconque des revendications 1 à 10 comprenant de plus un polymère épaississant associatif ou non associatif.
